# EUROPEAN PATENT APPLICATION

(11) **EP 3 693 058 A1**
(43) Date of publication of application: **12.08.2020**
(21) Application number: 17868499.9
(22) Date of filing: 03.10.2017
(51) Int. Cl.: A61N 1/40, H03B 5/00

(54) **WIDEBAND ELECTROMAGNETIC RESONATOR FOR THERAPEUTIC TREATMENT OF PATHOLOGICAL FOCI IN TISSUES OF AN ORGANISM, MEDICAL DEVICE FOR THERAPEUTIC TREATMENT AND METHOD OF THERAPEUTIC TREATMENT**

(71) Applicant: Zakrytoe Aktsionernoe Obschestvo "ES-Leasing", Moscow 117405 (RU)
(72) Inventor: SHMID, Alexander Viktorovich, Moscow 125310 (RU); BEREZIN, Andrey Alexandrovich, Moscow 125368 (RU)
(74) Representative: Eisenführ Speiser
(86) International application number: PCT/RU2017/000737
(87) International publication number: WO 2019/070144

(57) **Abstract**

Device for generating the Fermi-Pasta-Ulam (FPU) spectrum comprises a resonator for forming the FPU electromagnetic spectrum (FPU resonator), comprising a two-port generator for generating vibrations, each port of the generator comprising a coil, a capacitor and a nonlinear controlled electronic element, wherein said coils of the two-port generator have opposite winding and are counter-connected, have the same number and diameter of turns, and the turns of the one coil are disposed alternately between the turns of the other coil; the generator ports are connected such that to generate vibrations due to positive feedback between the generator ports; a power supply for supplying power to a connection point of the coils, and a switch on circuit connected to the two-port generator. There is also provided a medical device for therapeutic impact on lesions of different kinds in body tissues and a method of therapeutic impact on lesions in body tissues by resonance exposure of the FPU recurrence spectra in body tissues.

## Description

### Field of the Invention

The present invention relates to medical equipment, in particular, to a device, a medical device and a method of therapeutic impact on lesions in body tissues by radiating electromagnetic field having the structure of the Fermi-Pasta-Ulam (FPU) recurrence spectrum.

The medical device and method can be used for treating arrhythmia and inflammatory processes in the body, for harmonizing the work of organs and boosting the body defenses.

### Background of the Invention

An electronic device for electrical stimulation is known in the prior art (see e.g. RU 2135226 C1), which is intended for therapeutic non-invasive individually-dosed impact, determined from the group of selected zones and cyclical in time, on human skin areas by electrical pulses in order to exert general regulating influence on physiological systems of the body, and to provide analgesic effect.

The electrical stimulator comprises means for generating affecting pulses, means for generating modulation control signals, a controlled generator, a first and second electrodes, means for analysis of adaptation processes, a stimulation channel switching unit, 2N-2 electrodes, an electrode impact frequency generator, and a channel code former, wherein clock output of the means for generating affecting pulses is connected to clock inputs of the means for adaptation processes analysis and the electrode impact frequency generator, a control input of which is connected to a second control output of the means for adaptation processes analysis, and output is connected to a counting input of the channel code former; first and second outputs of i-th (i=I,N) group of outputs of means for switching stimulation channels are connected by i-th first and second electrodes of the group of 2N electrodes, respectively, and a second signal output of the means for generating output pulses is connected to a second signal input of the means for switching stimulation channels.

Human body is considered as a complex system, which is divided in accordance with the concepts of system analysis into multiple interrelated subsystems or interacting organs operating in total integrity.

In this context, percutaneous exposure at therapeutic non-invasive impact should be exerted on a set of selected zones to allow searching for effective impact on a group of organs of the human body.

A special need for percutaneous impact on a group of selected zones (actually a group of organs) appears also in the treatment of severe diseases (pathologies) associated with requirements of clinics, at which it is advisable to disturb the patient as little as possible, for example, when changing the bandage, and to provide a long-term stimulating impact on multiple zones of the skin simultaneously. The place of application of electrodes is chosen based on the response of the stimulated tissues or organs to the impact exerted.

After starting the stimulation process the effect of stimulating pulses is measured based on the response of tissues and organs of the human body. The feedback depth, i.e. the response time of the body, is regulated in the controlled generator by a second adjustment input.

A square-wave generator generates a sequence of square-wave pulses with frequencies selected by the attending physician and specified for each stimulation channel by setting of first adjustment inputs 11 - IN of the electrical stimulator. Duration of the generator pulses is varied by a modulator.

Output energy of the electrical stimulator is directly proportional to the duration of pulses supplied from output of the modulator to input of the power amplifier, and is measured from the blinking rate and luminescence intensity of the indicator. The generator also determines the clock frequency of the electrical stimulator.

The controlled generator specifies the length of series of stimuli and pauses between the series. To provide smooth rise and fall of energy of the stimulating pulses, output signals of the controlled generator are fed to means for generating modulation control signals, where they acquire a trapezoidal shape.

A limitation detector determines the amplitude of stimulation pulses. The amplitude of vibrations of output circuit of the amplifier is defined by the duration of pulses supplied from output of the modulator. Amplitude limiter of the means for generating modulation control signals actually sums signals of the controlled generator and the limitation detector.

As the body adapts to the stimulating impacts, vibrations in output circuit of the power amplifier of the means for generating affecting pulses are varied.

If there are pathological changes in the patient's body, stimulating pulses at the electrodes have an exponential decay with a time constant much smaller than that of a healthy body. The controlled generator produces series of pulses, in which duration of pauses between pulses greatly exceeds duration of pulses. Situations are even possible where a single short stimulating pulse is applied to the electrodes during one period.

As the stimulating pulses exert a therapeutic impact, after a certain period of time, defined by both the pathology level and the feedback depth set at the adjustment input, the response of the patient's body to the stimulating impact will be approaching the response of a healthy body or just stabilized. This is the condition of sufficiency of the exposure over the stimulation channel selected by the channel code former. This adaptation is monitored in the controlled generator and the means for analysis of adaptation processes.

Duration of the generator pulses is varied responsive to the feedback signal at input of the feedback signal former. If pathology is present in the selected points corresponding to the pair of electrodes, packs of stimulation pulses have duration less than the specified one. As the pathology is eliminated the influence on the generator over the feedback channel reduces, duration of stimulation pulse packs increases, i.e. duration of pulses of the time interval generator is restored.

A method of low-frequency electromagnetic therapy and device for implementing the method are known in the prior art (see e.g. RU 2164424 C1). The invention substantially relies on inducing resonance in the organs and systems with the aid of a weak electromagnetic field and harmonizing thereby the body functions.

A method of electromagnetic therapy comprises exposing biologically active points and biologically active zones to pulsed electromagnetic field with the intensity of 0.1 V/sq m. Electromagnetic pulses are radio pulses with repetition rate of packs of 0.1 - 100 Hz, resolution of 0.01 Hz and carrier frequency of 10 - 15 kHz. The device for low-frequency electromagnetic therapy comprises an antenna device, a power supply, a control keyboard, an LCD display, an antenna matching unit, a stabilizer whose input is connected to the power supply, a microprocessor controller connected by outputs to the control keyboard, the LCD display to show parameters of the executed treatment program and the antenna matching unit. The microprocessor controller is configured to store up to 1000 treatment programs and to form output pulses with a repetition frequency of 0.1 - 100 Hz, modulated with a frequency of 10 - 15 kHz.

Passive electrical properties of biological tissues are characterized by impedance, the magnitude of which is determined by capacitive and active conductivity with respective inductance of tissues. Active conductivity component at low frequencies is mainly due to the amount and electrolyte composition of interstitial fluid, while at high frequencies an additional contribution is made by the conductivity of cells. Since in the equivalent circuit of tissue the resistance of cells is connected in series with the capacitance of the cell membrane, the phenomenon of frequency dispersion of conductivity of biological tissues is observed. Possessing high dielectric properties and extremely low thickness, bilayer lipid membranes are characterized by high specific capacitance. High magnitude of charge capacity of membranes, and, consequently, capacitive properties of biological tissues are caused by considerable polarization capability of the membrane dielectric, depending on its relative dielectric permittivity. At high frequencies the polarization mechanisms are shut down with slowing the relaxation time, therefore, with increasing the frequency the capacitance of tissues should decrease just as in the case with increasing the dielectric permittivity.

In the low-frequency domain the impedance of tissues is determined mainly by their resistive properties. This domain includes tissues having a high conductivity, e.g. nervous tissue. The mid-range frequency domain includes tissues whose electric properties are determined by both resistive and capacitive properties, e.g. parenchymal organs. In the high-frequency domain electrical properties of tissues are capacitive, they are e.g. membranes and lipids. Slow polarization mechanisms in this frequency domain can lead to significant dielectric losses in tissues due to heating.

Therefore, a living cell can be presented as a resonant circuit with capacitance and resistance, where capacitance is determined by free radical reactions and antioxidant defense system, and resistance is determined by enzymatic oxidation.

Resonant circuit has a property of inductance, i.e. the ability to induce electrical current in another circuit or a closed conductor due to its magnetic moment. Generation of electromagnetic pulses from units to tens of Hz is a characteristic feature of normal functioning of various human organs.

Not only a cell can be presented in the form of a resonant circuit, but also higher levels of organization of living matter: tissues and organs with different predominance of glucose oxidation pathways, systems of organs and the whole body can be presented as an induced equilibrium system of resonant circuits. For example, the organ such as liver comprises both glucose oxidation pathways in equal proportion, which makes liver the key organ in the system of regulation of capacity and inductance of the body.

Circulatory system as such can be also presented as a cascade of closed conductors, from loops of capillaries to the greater and lesser circulation. Different impedance of venous and arterial blood creates conditions for mutual influence of organs on each other. Electrical properties of blood are determined by the amount of contained hemoglobin, oxygen, other cyclic compounds, protein and electrolyte composition, and the blood flow rate.

Thus, electromagnetic field in the framework of classical electrodynamics can integrate functioning of the whole body, creating and maintaining the specialization of different tissues. And the circulatory system is the intermediary through which regulation is performed.

Electromagnetic vibrations, inherent in tissues of a living body as such, are only partially dependent on vibrations existing outside the body. Although natural vibrations of the body are excited by vibrations of external electromagnetic fields, they are formed further again in the body in a specific form. Every organ and every cell has its own specific spectrum of vibrations, own specific characteristics of these vibrations (shape and appearance, as well as frequency). Maintaining these vibrations depends on the Q-factor of the resonator of cell, organ, tissue, or body as a whole. If the Q-factor of the resonator is broken or missing, there can appear incoherent, inadequate, abnormal electromagnetic vibrations. In the case where the self-regulation and healing mechanism existing in the body is unable to destruct these vibrations, a disease occurs.

Specific responses of human body to exposure to artificial electromagnetic field (EMF) were detected only at transition to ultra-low intensities of low frequencies in the range from 0.1 to 100 Hz EMF, when the strength of the field induced inside the body is significantly less than 0.1 V/cm.

When comparing the effects of artificial and natural low frequencies in the range of from 0.1 to 100 Hz EMF on a human it should also be taken into account that exposure to artificial EMFs is short, its duration is much less than human life, while effects of natural EMFs are being exerted continuously throughout life. In this regard, it can be expected that to obtain a therapeutic effect under the exposure to artificial electromagnetic field, its strength must be higher.

In accordance with the principle of reciprocity of antennas, any structure receiving EMF is also capable to emit EMF in the same frequency range.

Strong vibration of systems with weakly suppressed natural vibrations, if they are excited by relatively weak
external forces with a frequency equal to or nearly equal to the natural frequency of the system, is called resonance.

Therefore, resonance is induced in organs and systems with the aid of weak electromagnetic field and thereby functioning of the body is harmonized. Since diseases of organs may have different origins, it is necessary to use different systems of the body, i.e. use sets of frequencies, for treatment. Thus, a program of therapeutic impact comprises a set of frequencies, each of which works for a predetermined time, causing resonance in the desired organs and systems. Low-frequency electromagnetic therapy induces resonance phenomena, but the energy introduced into the body is so small that there is no overdose effect, and this is important, especially for treatment of cancer patients.

A device and method for inducing apoptosis of pathological cells and tissues are known in the prior art (see e.g. RU 2245728 C2).

Apoptosis is a morphologically distinct form of programmed death of cells, associated with vital processes of cells and playing an important role during development, homeostasis and in many diseases, including cancer, acquired immunodeficiency syndrome and neurodegenerative disorders, as well as with variable processes of survival of cells. Apoptosis occurs by activating the internal cell self-destruction program. Activation of self-destruction program is regulated by many different signals originating from both intracellular and external environment.

The method comprises exposing pathological cells and tissues to fixed and/or variable low-frequency electromagnetic fields with induction from 1 to 100 MT, frequency 1 to 1000 Hz. The device comprises means for generating constant and/or low-frequency alternating electromagnetic fields that permeate the work space. The method and device can influence cellular survival processes of living pathological cells by magnetic fields without harmful effects to healthy cells. In particular, the method comprises exposure to electromagnetic constant field (C-field) and electromagnetic low-frequency field (ELF-field) generated by this device.

The concept underlying the method is that CELF-fields affect cellular signals confirming the development of a pathological process inside pathological cells, i.e. signals on redox potential of free radicals, and thereby restore the cell survival processes, i.e. directly or indirectly stimulate apoptosis by modifying the expression of p53 gene.

The reason for selectively inducing apoptosis in pathological cells (i.e. cancer cells) by C- and ELF-fields can reside in altered electric behavior of pathological cells compared to healthy cells. For these reason the CELF-fields can cause, directly or indirectly, signal programmed death of cells - apoptosis, in vitro and in vivo, without causing as adverse effect.

There were used sequences of magnetic C-fields with different magnetic induction, amplitude-modulated, and with
application of magnetic ELF-fields. The use of modulated fields is necessary to achieve the optimal condition for the conversion of singlet-triplet state of spin required for the processes of recombination of free radicals.

Preferably, software should set C- and ELF-fields in accordance with the total magnetic induction from 1 to 30 MT and the C/ELF-field ratio in the range from 0.1 to 10, respectively, and in some special options, with the total magnetic induction from 1 to 10 MT and the C/ELF-field ratio in the range from 0.5 to 5, respectively.

A disadvantage of therapeutic effect of the conventional methods is the fact that the radiation is produced in a narrow range of frequencies. However, the body is a distributed self-vibrating system with many degrees of freedom, and external influence on it in a narrow frequency range results in that the body tissues in accordance with properties of nonlinear oscillatory systems alter their resonant characteristics in order to minimize the external influence, which leads to a decrease in therapeutic effect.

### Theoretical Justification basis of the Present Method

In 1954, Fermi, Pasta and Ulam attempted to show numerically the inevitable transition of a nonlinear system to a state with equally distributed energy from the state of monomode excitation. The system simulated by them is a chain of N particles of the same mass, connected by springs, with linear and nonlinear terms in the interaction force.

Based on theoretical considerations, Fermi, Pasta and Ulam expected that the energy, initially concentrated in the low-frequency mode qo, will be redistributed as the result of nonlinear interaction among the other modes, reproducing thereby the transition to equilibrium. To their surprise, the results were the opposite. Energy was not redistributed in any uniform manner, but rather returned to its original mode (up to 98%) after long periods of time. Even more impressive was the fact that the time of return even reduced with increasing energy/nonlinearity coefficient. The explanation that this is numerical error was refuted later by numerical experiments, and improved accuracy and greater integration times allowed observing the return to 99% of the initial energy.

Theoretical model of the nonlinear benchmark acoustic and electric dynamics of DNA formed the basis for the creation of a family of radio electronic devices, generators of solitary wave packets in the form of the Fermi-Pasta-Ulam recurrence (FPU generators), designed to generate electromagnetic waves (solitons) having a characteristic spatial-temporal structure of the FPU recurrence. The FPU recurrence spectrum is expressed in a periodic transition of oscillatory structure from an ordered state to pseudo chaotic one and back again. In the ordered state the initial form of a wave packet and its space-time spectrum are completely repeated recurred.

An important feature of the FPU generator is a spatial-temporal structure of its field, which corresponds to the vibration structure of the DNA molecule. The result of mathematical simulation shows that the structure of the DNA molecule corresponds to the FPU resonator.

This property allows using the generator in experiments on studying natural vibrations in the DNA products and information interaction of biological systems mentioned above. First models of such generators were designed by one of present inventors in 1988 - 1989.

A basic configuration of the generator comprises a FPU resonator in the form of two long lines with nonlinear elements (tunneling diodes for example) connected to them (see e.g. Longren K. "Experimental studies of solitons in nonlinear transmission lines with dispersion", "Solitons in action", Moscow, Mir, 1981, pp. 138 - 162).

The present method is based on the use of the general fundamental property of all distributed nonlinear oscillatory systems, including biological ones, the FPU recurrence phenomenon spectrum, according to which any dynamic system with many degrees of freedom has its own quasi-periodic recurrence of dynamics of Fourier spectrum pattern of initial disturbance of the system to its original form.

Quasi-periodic transition between determinate and quasi-chaotic state of the FPU spectrum ensures the establishment of a broadband resonance of electromagnetic FPU recurrence spectrum of an external generator with the FPU spectra in body tissues. This causes destruction of autonomy of the pathological biochemical process, and as a consequence, a decrease in its energy, which ultimately leads to elimination of the lesion from the body, or in other cases provides recovery of the heart rate to normal state.

This is the fundamental distinction of the mechanism of action of the FPU generator from currently used devices.

To generate the broadband spectrum a circuit for forming the FPU recurrence is required, which includes an element with a periodic structure. An example of such element is a coil, because a coil is a periodic structure or a helical slow-wave system. A condition is also necessary for occurrence of standing wave as in the canonical FPU chain with fixed ends. In the present two port or symmetric generator this is provided by periodic switching the generator ports and opposite winding of the coils. In addition, periodic structures of crystal lattices of the coil material also add harmonics to the high frequency portion of the spectrum.

FPU spectrum "breathes". "Breath" of the FPU spectrum is expressed in pumping the energy from low-frequency portion of the spectrum to the high-frequency one and back. This is the main property of the FPU spectrum. Interaction of different FPU spectra is impossible without this energy pumping.

### SUMMARY OF THE INVENTION

The object of the present invention is to provide a device for generating the Fermi-Pasta-Ulam (FPU) recurrence spectrum, which is broadband, for resonance interaction with FPU recurrence spectra existing in body tissues.

Another object of the present invention is to provide a medical device for therapeutic impact on lesions in body
tissues, comprising a device for generating the Fermi-Pasta-Ulam (FPU) spectrum for resonance exposure of distributed dynamic systems of the body by a generator of electromagnetic field vibrations having the structure of the FPU recurrence spectrum, which provides suppression of pathological processes in body tissues by resonance interaction with FPU recurrence spectra in body tissues and increases the extent of synchrony of dynamics of elements in associated dynamical systems of the body.

One more object of the present invention is to provide a method for generating the Fermi-Pasta-Ulam (FPU) spectrum for resonance exposure of distributed dynamic systems of the body by a generator for generating electromagnetic field vibrations having the structure of the FPU recurrence spectrum, which suppresses pathological processes in body tissues by resonance interaction with FPU recurrence spectra in body tissues and increases the extent of synchrony of dynamics of elements in associated dynamical systems of the body.

Resonance interaction generally occurs between identical molecules if one of them is in electronically excited state. The interaction is caused by the fact that when molecules are approaching each other excitation is transferred from one molecule to the other and back. As a result, both molecules, rather than one, appear to be in the excited state, and the energy level of this state is now different from that of individual molecule.

Resonance interaction plays a very important role in the systems, causing movement of excitation energy.

The object is attained in a device for generating the Fermi-Pasta-Ulam (FPU) spectrum, the device comprising:
a resonator for forming the FPU electromagnetic spectrum (FPU resonator), comprising:
a two-port generator for generating vibrations, each port of the generator comprising a coil, a capacitor and a nonlinear controlled electronic element;
wherein said coils of the two-port generator have opposite winding and are counter connected, have the same number and diameter of turns, and the turns of the one coil are disposed alternately between the turns of the other coil;
wherein the generator ports are connected to provide generation of vibrations due to positive feedback between the generator ports;
a power supply for supplying power to a connection point of the coils;
a switch on circuit connected to the two-port generator.

Advantageously, the nonlinear controlled electronic element can be an element selected from the group consisting of a bipolar transistor, a thyristor, a field-effect transistor, a chip, and a radio tube.

Preferably, the device further comprises a secondary resonator that is in electromagnetic coupling with the coils of the FPU resonator and designed to amplify power emitted by the FPU resonator, comprising:
two secondary counter-connected coils with opposite winding, having the same diameter of the turns, wherein the turns of the one coil are disposed alternately between the turns of the other coil, and terminals of said secondary coils are connected to a load to form a closed loop;
wherein the number of the turns of the secondary coils is twice as many as the number of the turns of the primary coils, and the turns of the secondary coils are disposed in pairs between the turns of the primary coils, the diameter of the turns of the secondary coils being equal to the diameter of the turns of the primary coils.

Advantageously, the turns of the primary coils are disposed close to each other without a gap.

It is also advantageous that the turns of the primary and secondary coils are disposed close to each other without a gap.

Preferably, the primary and secondary coils are made of wire having square or round cross section.

Advantageously, the diameter of the primary coils is within the range of 20 - 25 mm.

Advantageously, the wire diameter of the primary coils is 2 - 2.5 times the wire diameter of the secondary coils.

Advantageously, the wire diameter of the primary coils is from 1.8 to 2.2 mm.

Advantageously, the wire diameter of the secondary coils is from 0.8 to 1.2 mm.

Preferably, the load is a high-power diode.

Preferably, the device further comprises a two-port generator operation light indicator coupled to one of the generator ports.

Advantageously, the operation light indicator is a light emitting diode.

According to a second embodiment of the invention there is provided a device for generating the Fermi-Pasta-Ulam (FPU) spectrum, the device comprising:
a resonator for forming a high-frequency portion of the FPU electromagnetic spectrum, comprising:
two connected coils with opposite winding, having the same number and diameter of turns, wherein the turns of the one coil are disposed alternately between the turns of the other coil;
a circuit for forming a low-frequency portion of the FPU electromagnetic spectrum, comprising a nonlinear element and a low-ohmic resistor and a super-capacitor, connected in parallel and coupled to output of the nonlinear element;
wherein the other output of the nonlinear element is connected to the other terminal of said one of the coils;
a circuit for adjusting the frequency of the low-frequency portion of the spectrum in accordance with the patient's heart rate, comprising a capacitor and a controlled high-ohmic resistor, one output of the resistor is connected to a control electrode of the nonlinear element and the other output of the resistor is connected to one terminal of the second coil;
a led low-frequency vibration indicator coupled to the control electrode of the nonlinear element and designed to indicate short-term switching-on the resonator with a frequency close to the pulse rate of 1-1.5 Hz;
a power supply for supplying power to the coils;
a switch mounted at output of the power supply.

Preferably, the nonlinear controlled electronic element is an element from the group consisting of a bipolar transistor, a thyristor, a field-effect transistor, a chip, and a radio tube.

Advantageously, the device further comprises a core of dielectric material, on which the coils are arranged.

Preferably, turns of the coils are disposed close to each other without a gap.

Advantageously, the coils are made from a square or round wire.

Advantageously, the diameter of the coils is within the range of 20 - 25 mm.

Preferably, the wire diameter of the coils is from 1.8 to 2.2 mm.

In a preferred embodiment, a device for generating the Fermi-Pasta-Ulam (FPU) spectrum comprises:
a resonator for forming the FPU electromagnetic spectrum, comprising:
a two-port generator for generating vibrations, each port of the generator comprising a coil and connected in series a capacitor and a transistor;
wherein said coils of the two-port generator have opposite winding and are counter connected, have the same number and diameter of turns, and the turns of the one coil are disposed alternately between the turns of the other coil, and a terminal of one of the generator coils is coupled to the collector of the transistor of the one generator port, and a terminal of the other generator coil is coupled to the collector of the transistor of the other generator port;
wherein the generator ports are connected such that to provide generation of vibrations due to positive feedback between the generator ports;
a power supply for supplying power to a connection point of the coils;
two secondary counter-connected coils with opposite winding for amplifying power of the FPU resonator, having the same diameter of turns, wherein the turns of the one coil are disposed alternately between the turns of the other coil, and terminals of said secondary coils are coupled to a load to form a closed loop;
wherein the number of the turns of the secondary coils is twice as many as the number of the turns of the primary coils, and the turns of the secondary coils are disposed in pairs between the turns of the primary coils to provide electromagnetic coupling, the diameter of the turns of the secondary coils being equal to the diameter of the turns of the primary coils;
a switch on circuit comprising connected in series a resistor and a switch, wherein output of the resistor is coupled to the collector of the transistor of one of the generator ports, and output of the switch is coupled to the base of the same transistor.

In another preferred embodiment, a device for generating the Fermi-Pasta-Ulam (FPU) spectrum comprises:
a resonator for forming a high-frequency portion of the FPU electromagnetic spectrum, comprising:
two connected coils with opposite winding, having the same number and diameter of turns, wherein the turns of the one coil are disposed alternately between the turns of the other coil;
a circuit for forming a low-frequency portion of the FPU electromagnetic spectrum, comprising a transistor and connected in parallel a low-ohmic resistor and a super-capacitor, wherein output of the resistor is coupled to the emitter of the transistor, and the other output of the resistor is grounded, and the collector of the transistor is coupled to the other terminal of said one of the coils;
a circuit for adjusting the frequency of the low-frequency portion of the spectrum in accordance with the patient's heart rate, comprising a capacitor and a controlled high-ohmic resistor, one output of which is coupled to the base of the transistor of the resonance circuit, and the other output is coupled to one of terminals of the second coil;
a led low-frequency vibration indicator coupled to the base of the transistor and designed to indicate the short-term switching-on the high frequency generator at a low frequency close to the pulse rate of 1 - 1.5 Hz;
a power supply to which a terminal of one of the coils is connected;
a switch mounted at output of the power supply.

According to a second aspect of the invention, there is provided a medical device for therapeutic impact on lesions of different kinds in body tissues by resonance exposure of the FPU recurrence spectra in body tissues, the device comprising:
a housing of a non-magnetic material for placing on the patient's body, said housing enclosing in its cavities:
a device for generating the Fermi-Pasta-Ulam (FPU) spectrum according to claim 1 or 14;
a microprocessor to which said device is connected;
a temperature sensor for monitoring the temperature in the lesion area, connected with the microprocessor.

According to a second embodiment, there is provided a medical device for therapeutic impact at arrhythmia by resonance exposure of the FPU recurrence spectra existing in the cardiac muscle, comprising:
a housing of a non-magnetic material for placing on the patient's body, said housing enclosing in its cavities:
a device for generating the Fermi-Pasta-Ulam (FPU) spectrum according to claim 14;
a microprocessor to which said device is connected;
a pulse sensor adapted to be fixed on the patient's wrist and connected with the microprocessor.

According to a third embodiment, a medical device for therapeutic impact on lesions in body tissues by resonance exposure of the FPU recurrence spectra in body tissues, the device comprises:
a housing of a non-magnetic material for placing on the patient's body, said housing enclosing in its cavities:
the device for generating the Fermi-Pasta-Ulam (FPU) spectrum according to claim 14;
a microprocessor to which said device is connected;
a temperature sensor for monitoring the body temperature in the lesion area, connected to the microprocessor.

According to a thirst aspect of the invention, there is provided a method of therapeutic impact on lesions in the body by electromagnetic radiation having the form of the Fermi-Pasta-Ulam (FPU) spectrum for resonance exposure of the FPU recurrence spectra in body tissues, the method comprising:
using the medical device according to claim 22 or 24 for generating the FPU spectrum for resonance exposure on the FPU recurrence spectra in body tissues;
fixing the medical device on the patient's body in the lesion area subject to therapeutic impact;
exposing the lesion area to electromagnetic radiation of the FPU spectrum with a frequency ranging from 3 Hz to 650 MHz depending on the lesion type during 5 to 30 minutes;
monitoring the body temperature and judging whether therapeutic effect has been attained from increasing the body temperature in the lesion area by 0.4 - 2°C.

According to a second embodiment, a method of therapeutic impact at cardiac arrhythmia by electromagnetic radiation of the Fermi-Pasta-Ulam (FPU) spectrum for resonance exposure of the FPU recurrence spectra in the cardiac muscle, the method comprises:
using the medical device according to claim 23 for generating the FPU spectrum for resonance interaction with the FPU recurrence spectra in the cardiac muscle;
fixing the medical device on the patient's body in the heart region;
fixing a pulse sensor on the patient's wrist;
exposing the heart region to electromagnetic radiation of the FPU spectrum during 5 to 30 minutes with a frequency of the exposure in the range of 3 Hz to 650 MHz;
setting the resistance of the controlled high-ohmic resistor in accordance with the required pulse rate;
monitoring the pulse from the blinking frequency of the led low-frequency vibration indicator;
simultaneously monitoring the heart cardiogram and judging whether therapeutic effect has been attained from the heart cardiogram.

In accordance with the theory of resonant interaction between Fermi-Pasta-Ulam recurrences, the FPU recurrence energy of healthy tissues increases due to decrease in the FPU recurrence energy of tissues with pathology, which as a result significantly reduce their intensity or disappear altogether.

The present device provides forced synchronization of electromagnetic vibrations generated by the generator and characteristic to normal tissue ones with natural vibrations in body tissues. Due to the fact that lesions in tissues have oscillatory parameters different from those of normal tissues, at synchronization the energy of pathological vibrations in body tissues decreases and this causes a therapeutic effect.

### Brief Description of the Drawings

The invention is further explained by description of preferred embodiments with reference to the accompanying drawings, in which:
Fig. 1 shows an electric circuit of a device for generating FPU spectrum for resonance interaction with the FPU recurrence spectra in body tissues, according to the invention;
Fig. 2 shows schematically turns of coils of a primary and secondary resonator;
Fig. 3 shows an electric circuit of a device for generating the FPU spectrum, comprising a secondary resonator for amplifying the FPU electromagnetic spectrum of a primary resonator;
Fig. 4 shows the arrangement of turns of coils of a secondary resonator between turns of coils of a primary resonator;
Fig. 5 shows an electric circuit of a second embodiment of a device for generating the FPU spectrum, according to the invention;
Fig. 6 shows a medical device for generating the FPU spectrum for resonance interaction with the FPU recurrence spectra in body tissues, according to the invention;
Fig. 7 shows a second embodiment of a medical device for generating the FPU spectrum for resonance interaction with the FPU recurrence spectra in body tissues, according to the invention.

### Description of Preferred Embodiments

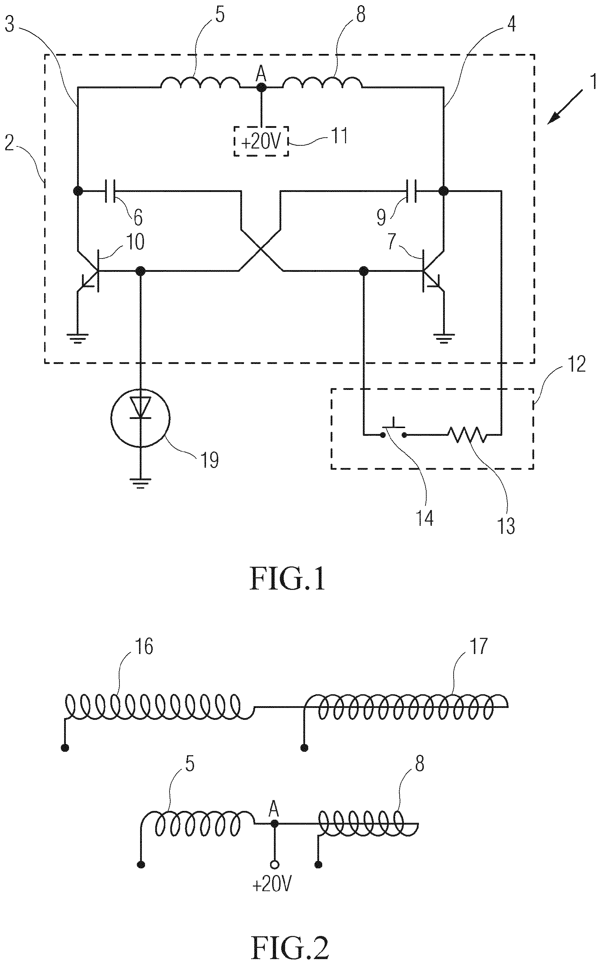

A device 1 (Fig. 1) for generating the Fermi-Pasta-Ulam (FPU) spectrum comprises a resonator 2 for forming the FPU electromagnetic spectrum (FPU resonator), comprising a two-port generator for generating vibrations, each port 3, 4 of which comprises a coil, a capacitor and a nonlinear controlled electronic element, in particular, the port 3 comprises a coil 5, a capacitor 6 and a nonlinear controlled electronic element 7, and the port 4 comprises a coil 8, a capacitor 9 and a nonlinear controlled electronic element 10.

The nonlinear controlled electronic element 7 or 9 can be an element selected from the group consisting of a bipolar transistor, a thyristor, a field-effect transistor, a chip, and a radio tube. This preferred embodiment uses a transistor.

The coils 5 and 8 of the two-port generator have opposite winding and are counter-connected as shown schematically in Fig. 2; they have the same number and diameter of turns. In the embodiment, the coils 5 and 8 have six turns, but there can be eight, or ten, or another number of turns. In the described structure, the turns of the one coil 5 are disposed alternately between the turns of the other coil 8.

Ports 3 and 4 of the two-port generator are connected such that to ensure generation of vibrations by the generator due to positive feedback between the ports 3 and 4.

The device 1 comprises a power supply 11, e.g. of 20 V DC voltage, which supplies power to connection point A of the coils 5 and 8.

In the described preferred embodiment, a terminal of one coil 5 is connected to the collector of a transistor 10 of one port 3 of the generator, while a terminal of the other coil 8 is connected to the collector of a transistor 7 of the other port 4.

The device 1 comprises also a trigger circuit 12, which comprises in this embodiment connected is series a resistor 13 and a switch 14; output of the resistor 13 is connected to the collector of the transistor 7 of the generator port 3, and output of the switch 14 is connected to the base of the same transistor 7.

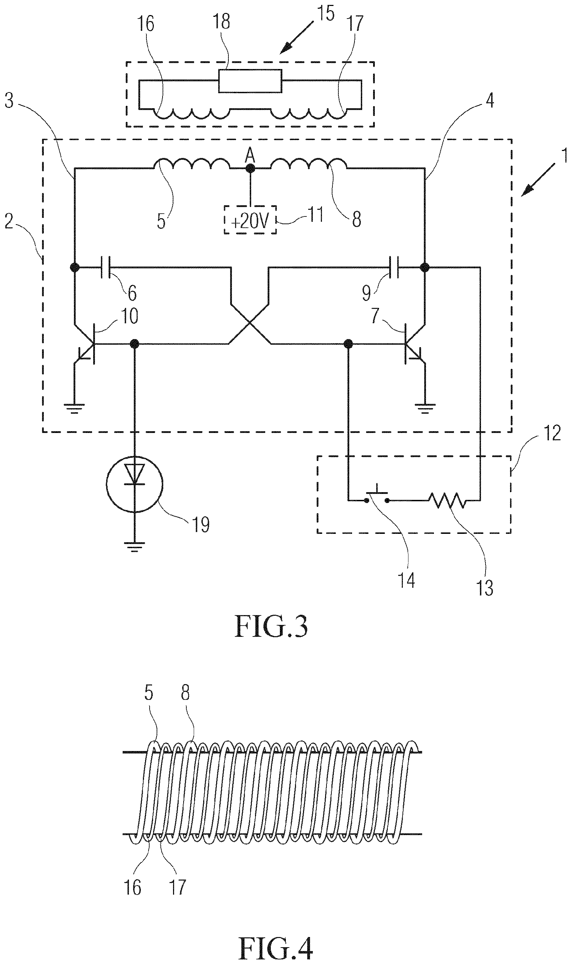

The device 1 preferably comprises a secondary resonator 15 (Fig. 3) that is in electromagnetic coupling with coils of the FPU resonator 1 and adapted to amplify power of the primary FPU resonator.

The secondary resonator 15 is formed e.g. of two secondary counter-connected coils 16, 17 with opposite winding, having the same diameter of turns, where the turns of the one coil 16 are disposed alternately between the turns of the other coil 17. Terminals of the secondary coils 16, 17 are connected to a load 18 to form a closed loop.

The number of the turns of the secondary coils 16 and 17 is twice as many as the number of the turns of the primary coils 5 and 8, and the turns of the secondary coils 16 and 17 are disposed in pairs between the turns of the primary coils 5 and 8, and the diameter of the turns of the secondary coils 16 and 17 is equal to the diameter of the turns of the primary coils 5 and 8.

The generator ports 3, 4 switch the direction of current in the resonators 1 and 15.

The turns of the primary coils 5 and 8 are disposed close to each other without a gap. If the secondary resonator 15 is available, the turns of the primary and secondary coils 5, 16, 17 and 8 are disposed close to each other without a gap (Fig. 4).

Coils 5, 8, 16, 17 are made of square or round wire.

Preferably, the diameter of coils 5 and 8 of the primary resonator 1 is within the range of 20 - 25 mm.

The wire diameter of the coils 5 and 8 is 2 - 2.5 times the wire diameter of the secondary coils 16 and 17; the wire diameter of the primary coils 5 and 8 in this embodiment is within the range of from 1.8 to 2.2 mm, while the wire diameter of the secondary coils 16, 17 is from 0.8 to 1.2 mm.

The load 18 of the secondary resonator 15 is a high-power diode, e.g. with direct current of 5 A and higher. The diode, as nonlinear element, influences the spectrum of the generator vibrations; additional harmonics appear, i.e. the set of harmonics increases and the spectrum is enriched, in particular, due to the nonlinearity of PN junction.

The device 1 further comprises a two-port generator operation light indicator 19 coupled to one port 3 of the generator; the light indicator 19 is a light-emitting diode (led).

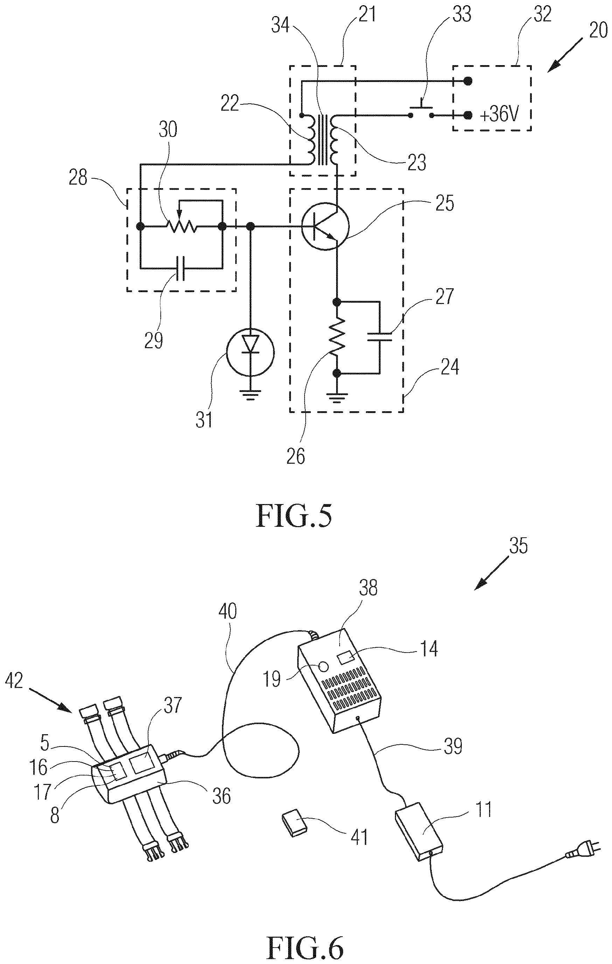

According to a second embodiment, a device 20 (Fig. 5) for generating the Fermi-Pasta-Ulam (FPU) spectrum comprises a resonator 21 for generating a high-frequency portion of the FPU electromagnetic spectrum (FPU resonator), comprising two counter or accordant connected coils 22, 23 with opposite winding, having the same number and diameter of turns, where the turns of the one coil 22 are alternately disposed between the turns of the other coil 23.

The device 20 further comprises a circuit 24 for generating a low-frequency portion of the FPU electromagnetic spectrum, comprising a nonlinear element 25 and connected in parallel a low-ohmic resistor 26 and a super-capacitor 27, both connected to output of the nonlinear element 25.

In the described embodiment, the nonlinear controlled electronic element 25 can be an element selected from the group consisting of a bipolar transistor, a thyristor, a field transistor, a chip, and a radio tube. In the preferred embodiment, the element is a transistor. The other output of the nonlinear element 25 is coupled to the other terminal of said one of the coils 23.

The device 20 further comprises a circuit 28 for adjusting the frequency of the low-frequency portion of the spectrum in accordance with the patient's heart rate. The circuit 28 comprises a capacitor 29 and a controlled high-ohmic resistor 30, an output of which is coupled to a control electrode of the nonlinear element 25, while the other output is connected to one of terminals of the second coil 22.

The device 20 further comprises a led low-frequency vibration indicator 31 coupled to the control electrode of the nonlinear element 25, in this case to the transistor base. The indicator 31 is intended to indicate short-term switching-on the resonator 21, which operates with a frequency close to the pulse rate of 1 - 1.5 Hz.

The device 20 in the described embodiment further comprises a power supply 32 to supply power to the coils 22 and 23, and a switch 33 mounted at output of the power supply 32.

The device 20 further comprises a core 34 of a dielectric material, on which coils 22 and 23 are mounted. Turns of the coils 22 and 23 are disposed close to each other without a gap.

Preferably, the coils 22 and 23 are made of square or round wire. The diameter of the coils 22 and 23 is preferably 20 - 25 mm, and the wire diameter of the coils is preferably from 1.8 to 2.2 mm.

According to a second aspect of the present invention there is provided a medical device 35 (Fig. 6) for therapeutic impact on lesions in body tissues by resonance exposure of the FPU recurrence spectra in body tissues, comprising: a housing 36 of a nonmagnetic material for placing on the patient's body. The housing 36 encloses in its cavities coils 5, 8, 16, 17 of a FPU resonator 2 of a device 1 for generating the Fermi-Pasta-Ulam (FPU) spectrum and a microprocessor 37, to which a switch 14 of a trigger circuit of the device 1 is connected.

A two-port generator is accommodated in a separate housing 38. On the housing 38 there is provided a device operation light indicator 19 and a switch 14 of the trigger circuit 12, both electrically connected to the microprocessor 37. The coils 5 and 8 are connected to the power supply 11 with wires 39 and 40.

In a set, the medical device 35 further comprises a temperature sensor 41 for monitoring the temperature in the lesion region, coupled to the microprocessor 37.

The housing 37 comprises means 42 to secure the device on the patient's body.

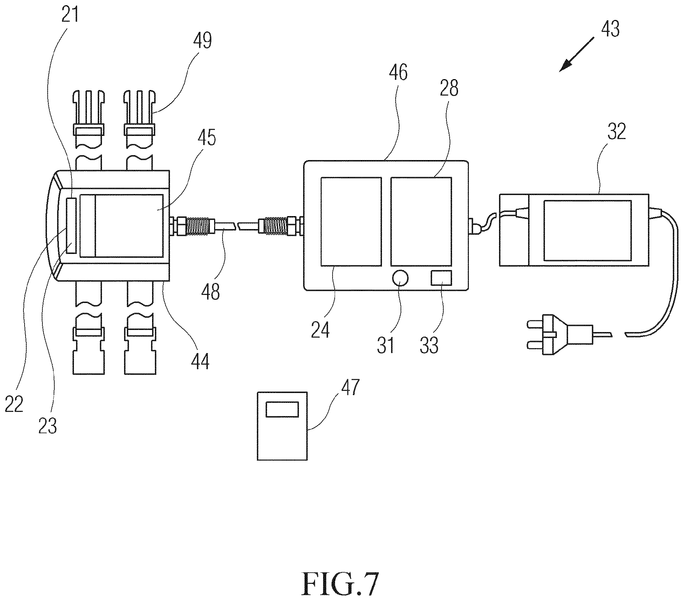

According to a second embodiment, there is provided a medical device 43 (Fig. 7) for therapeutic impact at arrhythmia by resonance exposure on the FPU recurrence spectra in the cardiac muscle. The medical device comprises a housing 44 of a nonmagnetic material for placing on the patient's body. The housing 44 encloses, in its cavities, coils 22, 23 of a resonator 21 and a microprocessor 45, to which a switch 33 of the device 20 for generating the Fermi-Pasta-Ulam (FPU) spectrum is coupled.

The medical device 43 further comprises an additional housing 46 accommodating a circuit 24 for forming a low-frequency portion of the FPU electromagnetic spectrum and a circuit 28 for adjusting the frequency of the low-frequency portion of the spectrum in accordance with the patient's heart rate. Front panel of the housing 46 comprises a low-frequency vibration led indicator 31 and a switch 33.

In a set, the medical device 42 comprises a pulse sensor 47 to be fixed on the patient's wrist, coupled to the microprocessor 45.

Fig. 7 also shows a power supply 32 connected by wires 48 to coils 22, 23 of the resonator 20. The housing 44 comprises means 49 to secure the device on the patient's body in the heart region.

A second possible embodiment of a medical device 43 (not shown) for therapeutic impact on lesions in body tissues by resonance exposure of the FPU recurrence spectra in body tissues, comprises: a housing of a non-magnetic material for placing on the patient's body, the housing enclosing, in its cavities, coils 22 and 23 of the device 20 for generating the Fermi-Pasta-Ulam (FPU) spectrum and a microprocessor to which the device 20 is connected; the medical device set includes a temperature sensor for monitoring the body temperature in the lesion region, connected to a microprocessor.

In all embodiments, the microprocessor can be used to switch the medical devices on and off, e.g. upon attaining a predetermined therapeutic effect.

The medical device operates in the following manner.

Operation of the device is initiated by supplying positive voltage to the transistor base upon pressing the start button. During operation the led should light permanently to show the presence of vibrations in the generator, because even at the turned-on power source the generator may not work due to various reasons.

Time for establishing the operation mode of the medical device upon turning-on should not exceed 1 min. Establishment of the operation mode is accompanied by turning-on light indication.

The medical device is intended for use in hospitals, specialized clinics and hospitals, medical centers and at home in normal climatic conditions that are characterized by the following values: ambient temperature from 15°C to 25°C C; relative humidity from 45% to 75%; atmospheric pressure from 97.3 kPa to 105.3 kPa (730 mmHg to 790 mmHg).

On the housing there can be provided a toggle to switch the operation mode, since the generator may have two operation modes: more intense and less intense, marked HIGH/LOW.

The principle of operation is based on the impact of the FPU spectrum on various pathological processes that occur in the cardiac muscle. Owing to the quasi-periodical transition between regular and chaotic vibrations of the generator there occurs a nonlinear FPU resonance with own oscillatory processes in the myocardium. Duration of one procedure is 5-20 minutes, depending on the individual recommendations.

A method of therapeutic impact on lesions in the body by electromagnetic radiation of the Fermi-Pasta-Ulam (FPU) spectrum for resonance exposure of the FPU recurrence spectra in body tissues is accomplished in the following manner.

The medical device 35 for generating the FPU spectrum for resonance exposure of the FPU recurrence spectra in body tissues is fixed on the patient's body in the region of the lesion subject to therapeutic impact.

The medical device is switched on and the lesion region is exposed to electromagnetic radiation of the FPU spectrum with a frequency in the range of from 3 Hz to 650 MHz depending on the type of the lesion for 5 to 30 minutes.

At the same time, the body temperature is monitored, and the attainment of therapeutic effect is determined from increase in the body temperature in the lesion region by 0.4 - 2°C; to this end the body temperature in the lesion region is permanently measured by a temperature sensor 39. When the body temperature in the lesion region increases by 0.4 - 2 °C, the attainment of therapeutic effect is determined. Temperature increases always, but in healthy people the body temperature increased by not more than 0.1 -0.2°C, while if there is an inflammatory lesion the temperature under the radiation increases by 0.4 - 2°C.

The session generally lasts from 10 to 30 minutes. To achieve the therapeutic effect 5 - 6 exposure sessions are accomplished.

The procedures are recommended at identical time intervals within the period from 4 pm to 10 pm, but they are also possible in the morning and in the afternoon. The course of treatment lasts for 3 to 6 days depending on doctor's recommendations and the disease severity.

The therapeutic effect occurs already after the first session.

As mentioned above, healthy tissues and tissues with pathology, e.g. with inflammation, have different Fermi-Pasta-Ulam (FPU) radiation spectra, which was proven by analysis of their electromagnetic radiation spectrum. Moreover, the interaction of radiation spectra of these healthy tissues and tissues with pathology with external electromagnetic radiation of the Fermi-Pasta-Ulam (FPU) spectrum from the medical device occurs in different way. Specific exposure characteristics are experimentally chosen and recommended, however, they can be modified according to the individual characteristics of a particular patient.

The FPU spectrum energy from the medical device is transferred to the FPU spectrum energy of healthy tissues and tissues with pathology. It should be noted that according to statistics the mass of tissues having pathology, compared to the mass of healthy tissue, is not more than 10%, i.e. it is significantly less. Therefore, the FPU spectrum energy, captured by healthy tissue when entering the resonance, is many times greater than the FPU spectrum energy, captured by tissue with pathology when entering the resonance.

The FPU spectrum energy of healthy tissues increases. It can be said that the FPU spectrum energy of healthy tissues inhibits the FPU spectrum energy of diseased tissues. As the result the FPU spectrum energy of tissues with pathology reduces.

In one example, a method of therapeutic impact at cardiac arrhythmia by electromagnetic radiation of the Fermi-Pasta-Ulam (FPU) spectrum for resonance exposure of the FPU recurrence spectra in the cardiac muscle is accomplished in the following manner.

A medical device 43 (Fig. 7) is fixed on the patient's body in the heart region, and a pulse sensor 47 is secured on the patient's wrist.

The heart region is exposed to electromagnetic radiation of the FPU spectrum during 5 to 30 minutes.

The resistance of a controlled high-ohmic resistor 30 is set in accordance with the required pulse rate, and the pulse rate is monitored from blinking frequency of a led low-frequency vibration indicator 31. The pulse rate is needed to implement resonance exposure of the heart; the allowable range is within the medical range of heart rate.

The heart cardiogram is continuously monitored to determine the achievement of therapeutic effect from it.

The medical device is used for treatment of cardiac arrhythmias of different origin. The device provides forced synchronization of oscillatory processes in the myocardium or in other tissues of the body in a continuous mode within a predetermined time of the therapeutic impact.

The medical device relates to therapeutic devices operating preferably at frequencies from 3 Hz to 650 MHz.

Power consumed by the generator must be not more than 15 watts.

Indications for use:
- various inflammatory processes, including necrotizing inflammations after myocardial infarction, infiltrates in the lymph nodes (tonsillitis), sinusitis, periodontitis, and so on. Abnormal heart rhythm (arrhythmia);
- radiculitis, ischias, lumbago, intercostal neuralgia;
- post-traumatic inflammations after injuries, sprains and dislocations;
- effective at joint and rheumatic pains, arthritis;
- effective at hemorrhoids and prostatitis.

The exposure time is generally 20-25 minutes. The treatment cycle is 3-6 days. The most effective time for using the generator is from 4 pm to 10 pm.

### Example 1 of using the medical device

Patient D., 58 years, with sustained arrhythmia. The loss of pulse is determined at every third blow on average. The FPU generator was placed on the breast bone, and in five minutes
after starting the exposure the arrhythmia has decreased dramatically and disappeared altogether 10 minutes later. Five therapeutics sessions were conducted using the FPU generator with the exposure of 10 minutes. Within a few months after the therapy the observation did not reveal arrhythmia in the patient. Thus, the FPU generator was effective to eliminate arrhythmia.

### Example 2 of using the medical device

Patient A., 60 years. Attack of sciatica. Acute pain in the lumbar region hinders movements of the patient. The FPU generator was placed over the lumbar region. The exposure time was 35 minutes. During the exposure the pain almost disappeared and the patient was able to move freely. Four sessions were conducted with the exposure time of 35 minutes, after which the patient returned to work. Therefore, the FPU generator was effective for elimination of consequences of attack of sciatica.

### Industrial Applicability

The medical device and the method of therapeutic impact on lesions in body tissues by electromagnetic radiation having the structure of the Fermi-Pasta-Ulam (FPU) recurrence spectrum can be used for treatment of arrhythmia and inflammatory processes in the body, as well as for harmonization of functioning of organs and boosting the body's defenses.

## Claims

1. A device for generating the Fermi-Pasta-Ulam (FPU) spectrum, the device comprising:
a resonator for forming the FPU electromagnetic spectrum (FPU resonator), comprising:
a two-port generator for generating vibrations, each port of the generator comprising a coil, a capacitor and a nonlinear controlled electronic element;
wherein said coils of the two-port generator have opposite winding and are counter connected, have the same number and diameter of turns, and the turns of the one coil are disposed alternately between the turns of the other coil;
wherein the generator ports are connected to provide generation of vibrations due to a positive feedback between the generator ports;
a power supply for supplying power to a connection point of the coils;
a trigger circuit connected to the two-port generator.

2. The device according to claim 1, wherein the nonlinear controlled electronic element is an element selected from the group consisting of a bipolar transistor, a thyristor, a field-effect transistor, a chip, and a radio tube.

3. The device according to claim 1, further comprising a secondary resonator being in electromagnetic coupling with coils of the FPU resonator and designed to amplify power of the FPU resonator, comprising:
two secondary counter-connected coils with opposite winding, having the same diameter of turns, wherein the turns of the one coil are disposed alternately between the turns of the other coil, and terminals of said secondary coils are connected to a load to form a closed loop;
wherein the number of the turns of the secondary coils is twice as many as the number of the turns of the primary coils, and the turns of the secondary coils are disposed in pairs between the turns of the primary coils, the diameter of the turns of the secondary coils being equal to the diameter of the turns of the primary coils.

4. The device according to claim 1, wherein the turns of the primary coils are disposed close to each other without a gap.

5. The device according to claim 3, wherein the turns of the primary and secondary coils are disposed close to each other without a gap.

6. The device according to claim 1 or 3, wherein the primary and secondary coils are made of square or round wire.

7. The device according to claim 1, wherein the diameter of the primary coils is within the range of 20 - 25 mm.

8. The device according to claim 3, wherein the wire diameter of the primary coils is 2 - 2.5 times the wire diameter of the secondary coils.

9. The device according to claim 1, wherein the wire diameter of the primary coils is from 1.8 to 2.2 mm.

10. The device according to claim 3, wherein the wire diameter of the secondary coils is from 0.8 to 1.2 mm.

11. The device according to claim 3, wherein the load is a high-power diode.

12. The device according to claim 1, further comprising a two-port generator operation light indicator coupled to one of the generator ports.

13. The device according to claim 12, wherein the operation light indicator is a light emitting diode.

14. A device for generating the Fermi-Pasta-Ulam (FPU) spectrum, the device comprising:
a resonator for forming a high-frequency portion of the FPU electromagnetic spectrum, comprising:
two connected coils with opposite winding, having the same number and diameter of turns, wherein the turns of the one coil are disposed alternately between the turns of the other coil;
a circuit for forming a low-frequency portion of the FPU electromagnetic spectrum, comprising a nonlinear element and a low-ohmic resistor and a super-capacitor, connected in parallel and coupled to output of the nonlinear element;
wherein the other output of the nonlinear element is connected to the other terminal of said one of the coils;
a circuit for adjusting the frequency of the low-frequency portion of the spectrum in accordance with the patient's heart rate, comprising a capacitor and a controlled high-ohmic resistor, one output of the controlled resistor is connected to a control electrode of the nonlinear element and the other output of the controlled resistor is connected to one terminal of the second coil;
a led low-frequency vibration indicator coupled to the control electrode of the nonlinear element and designed to indicate short-term switching-on the resonator with a frequency close to the pulse rate of 1-1.5 Hz;
a power supply for supplying power to the coils;
a switch mounted at output of the power supply.

15. The device according to claim 14, wherein the nonlinear controlled electronic element is an element from the group consisting of a bipolar transistor, a thyristor, a field-effect transistor, a chip, and a radio tube.

16. The device according to claim 14, further comprising a core of dielectric material, on which the coils are arranged.

17. The device according to claim 14, wherein turns of the coils are disposed close to each other without a gap.

18. The device according to claim 14, wherein the coils are made of a square or round wire.

19. The device according to claim 14, wherein the diameter of the coils is within the range of 20 - 25 mm.

20. The device according to claim 14, wherein the wire diameter of the coils is from 1.8 to 2.2 mm.

21. A device for generating the Fermi-Pasta-Ulam (FPU) spectrum, the device comprising:
a resonator for forming the FPU electromagnetic spectrum, comprising:
a two-port generator for generating vibrations, each port of the generator comprising a coil and a capacitor and a transistor connected in series;
wherein said coils of the two-port generator have opposite winding and are counter connected, have the same number and diameter of turns, and the turns of the one coil are disposed alternately between the turns of the other coil, and a terminal of one of the generator coils is coupled to the collector of the transistor of one generator port, and a terminal of the other generator coil is coupled to the collector of the transistor of the other generator port;
wherein the generator ports are connected such that to provide generation of vibrations due to positive feedback between the generator ports;
a power supply for supplying power to a connection point of the coils;
two secondary counter-connected coils with opposite winding for amplifying power of the FPU resonator, having the same diameter of turns, wherein the turns of the one coil are disposed alternately between the turns of the other coil, and terminals of said secondary coils are coupled to a load to form a closed loop;
wherein the number of the turns of the secondary coils is twice as many as the number of the turns of the primary coils, and the turns of the secondary coils are disposed in pairs between the turns of the primary coils to provide electromagnetic coupling, the diameter of the turns of the secondary coils being equal to the diameter of the turns of the primary coils;
a switch on circuit comprising connected in series a resistor and a switch, wherein output of the resistor is coupled to the collector of the transistor of one of the generator ports, and output of the switch is coupled to the base of the same transistor.

22. A device for generating the Fermi-Pasta-Ulam (FPU) spectrum, the device comprising:
a resonator for forming a high-frequency portion of the FPU electromagnetic spectrum, comprising:
two connected coils with opposite winding, having the same number and diameter of turns, wherein the turns of the one coil are disposed alternately between the turns of the other coil;
a circuit for forming a low-frequency portion of the FPU electromagnetic spectrum, comprising a transistor and connected in parallel a low-ohmic resistor and a super-capacitor, wherein output of the resistor is coupled to the emitter of the transistor, and the other output of the resistor is grounded, and the collector of the transistor is coupled to the other terminal of said one of the coils;
a circuit for adjusting the frequency of the low-frequency portion of the spectrum in accordance with the patient's heart rate, the circuit comprising a capacitor and a controlled high-ohmic resistor, one output of the resistor is coupled to the base of the transistor of the resonance circuit, and the other output of the resistor is coupled to one of the terminals of the second coil;
a led low-frequency vibration indicator coupled to the base of the transistor and designed to indicate short-term switching-on the generator with a frequency close to the pulse rate of 1 - 1.5 Hz;
a power supply to which a terminal of one of the coils is connected;
a switch mounted at output of the power supply.

23. A medical device for therapeutic impact on lesions in body tissues by resonance exposure of the FPU recurrence spectra in body tissues, the device comprising:
a housing of a non-magnetic material for placing on the patient's body, said housing enclosing in its cavities:
a device for generating the Fermi-Pasta-Ulam (FPU) spectrum according to claim 1 or 14;
a microprocessor to which said device is connected;
a temperature sensor for monitoring the temperature in the lesion area, connected with the microprocessor.

24. A medical device for therapeutic impact at arrhythmia by resonance exposure of the FPU recurrence spectra in the cardiac muscle, the device comprising:
a housing of a non-magnetic material for placing on the patient's body, said housing enclosing in its cavities:
a device for generating the Fermi-Pasta-Ulam (FPU) spectrum according to claim 14;
a microprocessor to which said device is connected;
a pulse sensor adapted to be fixed on the patient's wrist and connected with the microprocessor.

25. A medical device for therapeutic impact on lesions in body tissues by resonance exposure of the FPU recurrence spectra in body tissues, the device comprising:
a housing of a non-magnetic material for placing on the patient's body, said housing enclosing in its cavities:
a device for generating the Fermi-Pasta-Ulam (FPU) spectrum according to claim 14;
a microprocessor to which said device is connected;
a temperature sensor for monitoring the body temperature in the lesion area and connected to the microprocessor.

26. A method of therapeutic impact on lesions in the body by electromagnetic radiation of the Fermi-Pasta-Ulam (FPU) spectrum for resonance exposure of the FPU recurrence spectra in body tissues, the method comprising:
using a medical device according to claim 23 or 25 for generating the FPU spectrum for resonance exposure on the FPU recurrence spectra in body tissues;
fixing the medical device on the patient's body in the lesion area subject to a therapeutic impact;
exposing the lesion area to electromagnetic radiation of the FPU spectrum with a frequency ranging from 3 Hz to 650 MHz depending on the lesion type during 5 to 30 minutes;
monitoring the body temperature and judging whether therapeutic effect has been attained from increasing the body temperature in the lesion area by 0.4 - 2°C.

27. A method of therapeutic impact at cardiac arrhythmia by electromagnetic radiation of the Fermi-Pasta-Ulam (FPU) spectrum for resonance exposure of the FPU recurrence spectra in the cardiac muscle, the method comprising:
using a medical device according to claim 24 for generating the FPU spectrum for resonance interaction with the FPU recurrence spectra in the cardiac muscle;
fixing the medical device on the patient's body in the heart region;
fixing a pulse sensor on the patient's wrist;
exposing the heart region to electromagnetic radiation of the FPU spectrum during 5 to 30 minutes with a frequency of the exposure in the range of 3 Hz to 650 MHz;
setting the resistance of the controlled high-ohmic resistor in accordance with the required pulse rate;
monitoring the pulse from the blinking frequency of the led low-frequency vibration indicator;
simultaneously monitoring the heart cardiogram and judging whether therapeutic effect has been attained from the heart cardiogram.

## Amended claims

### Amended claims under Art. 19.1 PCT

1. A device for generating the Fermi-Pasta-Ulam (FPU) spectrum, comprising:
a resonator for forming the FPU electromagnetic spectrum (FPU resonator), the resonator comprising:
a two-port generator for generating vibrations, each port of the generator comprising a coil, a capacitor and a nonlinear controlled electronic element;
wherein said coils of the two-port generator have opposite winding and are counter connected, have the same number and diameter of turns, and turns of one coil are disposed alternately between turns of the other coil;
wherein the generator ports are connected to provide generation of vibrations due to positive feedback between the generator ports;
a power supply for supplying power to a connection point of the coils;
a trigger circuit connected to the two-port generator.

2. A device according to claim 1, wherein the nonlinear controlled electronic element is an element selected from the group consisting of bipolar transistor, thyristor, field-effect transistor, chip, and radio tube.

3. A device according to claim 1, further comprising a secondary resonator being in electromagnetic coupling with coils of the FPU resonator and designed to amplify power of the FPU resonator, comprising:
two secondary counter-connected coils with opposite winding, having the same diameter of turns, wherein turns of one coil are disposed alternately between turns of the other coil, and terminals of said secondary coils are connected to a load to form a closed loop;
wherein the number of turns of the secondary coils is twice as many as the number of turns of the coils of the FPU resonator, and turns of the secondary coils are disposed in pairs between turns of the coils of the FPU resonator, the diameter of turns of the secondary coils being equal to the diameter of turns of the coils of the FPU resonator.

4. A device according to claim 1, wherein turns of the coils of the FPU resonator are disposed close to each other without a gap.

5. A device according to claim 3, wherein turns of the coils of the FPU resonator and secondary coils are disposed close to each other without a gap.

6. A device according to claim 1 or 3, wherein the coils of the FPU resonator and secondary coils are made of square or round wire.

7. A device according to claim 1, wherein the diameter of the coils of the FPU resonator is within the range of 20 - 25 mm.

8. A device according to claim 3, wherein the wire diameter of the coils of the FPU resonator is 2 - 2.5 times the wire diameter of the secondary coils.

9. A device according to claim 1, wherein the wire diameter of the coils of the FPU resonator is from 1.8 to 2.2 mm.

10. A device according to claim 3, wherein the wire diameter of the secondary coils is from 0.8 to 1.2 mm.

11. A device according to claim 3, wherein the load is a high-power diode.

12. A device according to claim 1, further comprising a two-port generator operation light indicator coupled to one of the generator ports.

13. A device according to claim 12, wherein the operation light indicator is a light emitting diode.

14. A device for generating the Fermi-Pasta-Ulam (FPU) spectrum, comprising:
a resonator for forming a high-frequency portion of the FPU electromagnetic spectrum, comprising:
two connected coils with opposite winding, having the same number and diameter of turns, wherein turns of one coil are disposed alternately between turns of the other coil;
a circuit for forming a low-frequency portion of the FPU electromagnetic spectrum, comprising a nonlinear element and a low-ohmic resistor and a super-capacitor, connected in parallel and coupled to output of the nonlinear element;
wherein the other output of the nonlinear element is connected to the other terminal of said one of the coils;
a circuit for adjusting the frequency of the low-frequency portion of the spectrum in accordance with the patient's heart rate, comprising a capacitor and a controlled high-ohmic resistor, one output of which is connected to a control electrode of the nonlinear element and the other output is connected to one terminal of the second coil;
a led low-frequency vibration indicator coupled to the control electrode of the nonlinear element and designed to indicate short-term switch the resonator with a frequency close to the pulse rate of 1-1.5 Hz;
a power supply for supplying power to the coils;
a switch mounted at output of the power supply.

15. A device according to claim 14, wherein the nonlinear controlled electronic element is an element from the group consisting of bipolar transistor, thyristor, field-effect transistor, chip, and radio tube.

16. A device according to claim 14, further comprising a core of dielectric material, on which the coils are arranged.

17. A device according to claim 14, wherein turns of the coils are disposed close to each other without a gap.

18. A device according to claim 14, wherein the coils are made of square or round wire.

19. A device according to claim 14, wherein the diameter of the coils is within the range of 20 - 25 mm.

20. A device according to claim 14, wherein the wire diameter of the coils is from 1.8 to 2.2 mm.

21. A device for generating the Fermi-Pasta-Ulam (FPU) spectrum, comprising:
a resonator for forming the FPU electromagnetic spectrum, comprising:
a two-port generator for generating vibrations, each port of the generator comprising a coil and connected in series a capacitor and a transistor;
wherein said coils of the two-port generator have opposite winding and are counter connected, have the same number and diameter of turns, and turns of one coil are disposed alternately between turns of the other coil, and a terminal of one of the generator coils is coupled to the collector of the transistor of one generator port, and a terminal of the other generator coil is coupled to the collector of the transistor of the other generator port;
wherein the generator ports are connected such that to provide generation of vibrations due to positive feedback between the generator ports;
a power supply for supplying power to a connection point of the coils;
two secondary counter-connected coils with opposite winding for amplifying power of the FPU resonator, having the same diameter of turns, wherein turns of one coil are disposed alternately between turns of the other coil, and terminals of said secondary coils are coupled to a load to form a closed loop;
wherein the number of turns of the secondary coils is twice as many as the number of turns of the coils of the FPU resonator, and turns of the secondary coils are disposed in pairs between turns of the coils of the FPU resonator to provide electromagnetic coupling, the diameter of turns of the secondary coils being equal to the diameter of turns of the coils of the FPU resonator;
a trigger circuit comprising connected in series a resistor and a switch, wherein output of the resistor is coupled to the collector of the transistor of one of the generator ports, and output of the switch is coupled to the base of the same transistor.

22. A device for generating the Fermi-Pasta-Ulam (FPU) spectrum, comprising:
a resonator for forming a high-frequency portion of the FPU electromagnetic spectrum, comprising:
two connected coils with opposite winding, having the same number and diameter of turns, wherein turns of one coil are disposed alternately between turns of the other coil;
a circuit for forming a low-frequency portion of the FPU electromagnetic spectrum, comprising a transistor and connected in parallel a low-ohmic resistor and a super-capacitor, wherein output of the resistor is coupled to the emitter of the transistor, and the other output of the resistor is grounded, and the collector of the transistor is coupled to the other terminal of said one of the coils;
a circuit for adjusting the frequency of the low-frequency portion of the spectrum in accordance with the patient's heart rate, comprising a capacitor and a controlled high-ohmic resistor, one output of which is coupled to the base of the transistor of the resonance circuit, and the other output is coupled to one of terminals of the second coil;
a led low-frequency vibration indicator coupled to the base of the transistor and designed to indicate short-term switching-on the generator with a frequency close to the pulse rate of 1 - 1.5 Hz;
a power supply to which terminal of one of coils is connected;
a switch mounted at output of the power supply.

23. A medical device for therapeutic impact on lesions in body tissues by resonance exposure of the FPU recurrence spectra in body tissues, comprising:
a housing of a non-magnetic material for placing on the patient's body, said housing enclosing in its cavities:
a device for generating the Fermi-Pasta-Ulam (FPU) spectrum according to claim 1 or 14;
a microprocessor to which said device is connected;
a temperature sensor for monitoring the temperature in the lesion area, connected with the microprocessor.

24. A medical device for therapeutic impact at arrhythmia by resonance exposure of the FPU recurrence spectra in the cardiac muscle, comprising:
a housing of a non-magnetic material for placing on the patient's body, said housing enclosing in its cavities:
a device for generating the Fermi-Pasta-Ulam (FPU) spectrum according to claim 14;
a microprocessor to which said device is connected;
a pulse sensor adapted to be fixed on the patient's wrist and connected with the microprocessor.

25. A medical device for therapeutic impact on lesions in body tissues by resonance exposure of the FPU recurrence spectra in body tissues, comprising:
a housing of a non-magnetic material for placing on the patient's body, said housing enclosing in its cavities:
a device for generating the Fermi-Pasta-Ulam (FPU) spectrum according to claim 14;
a microprocessor to which said device is connected;
a temperature sensor for monitoring the body temperature in the lesion area, connected to the microprocessor.

26. A method of therapeutic impact on lesions in the body by electromagnetic radiation of the Fermi-Pasta-Ulam (FPU) spectrum for resonance exposure of the FPU recurrence spectra in body tissues, comprising:
using a medical device according to claim 23 or 25 for generating the FPU spectrum for resonance exposure on the FPU recurrence spectra in body tissues;
fixing the medical device on the patient's body in the lesion area subject to therapeutic impact;
exposing the lesion area to electromagnetic radiation of the FPU spectrum with a frequency ranging from 3 Hz to 650 MHz depending on the lesion type during 5 to 30 minutes;
monitoring the body temperature and judging whether therapeutic effect has been attained from increasing the body temperature in the lesion area by 0.4 - 2°C.

27. A method of therapeutic impact at cardiac arrhythmia by electromagnetic radiation of the Fermi-Pasta-Ulam (FPU) spectrum for resonance exposure of the FPU recurrence spectra in the cardiac muscle, comprising:
using a medical device according to claim 24 for generating the FPU spectrum for resonance interaction with the FPU recurrence spectra in the cardiac muscle;
fixing the medical device on the patient's body in the heart region;
fixing a pulse sensor on the patient's wrist;
exposing the heart region to electromagnetic radiation of the FPU spectrum during 5 to 30 minutes with a frequency of the exposure in the range of 3 Hz to 650 MHz;
setting the resistance of the controlled high-ohmic resistor in accordance with the required pulse rate;
monitoring the pulse from the blinking frequency of the led low-frequency vibration indicator;
simultaneously monitoring the heart cardiogram and judging whether therapeutic effect has been attained from the heart cardiogram.
